# EUROPEAN PATENT APPLICATION

(11) **EP 2 452 677 A1**
(43) Date of publication of application: **16.05.2012**
(21) Application number: 12153532.2
(22) Date of filing: 29.09.2009
(51) Int. Cl.: A61K 9/20, A61K 9/28

(54) **Extended release dosage form of ropinirole**

(30) Priority: 29.09.2008 IN MU20912008; 29.09.2008 IN MU20922008
(62) Divisional of application: 09736685.0
(71) Applicant: Wockhardt Limited, Aurangabad 431210 (IN)
(72) Inventor: Nakhat, Premchand, 445001 Yavatmal (IN); Joshi, Venkatesh, 584103 Nagar Raichur (IN); Mandaogade, Prashant, 444604 Amravati (IN); Jain, Girish, Kumar, 10034 Delhi (IN)
(74) Representative: May, Mark Andrew

(57) **Abstract**

The present invention relates to extended release pharmaceutical compositions of ropinirole or salts thereof, comprising a core of ropinirole or salts thereof and outer mantle coating surrounding the core comprising one or more pharmaceutically acceptable excipients.

## Description

### Field of the Invention

There is provided an extended release tablet in tablet dosage form of ropinirole or salts thereof comprising a) an inner tablet of ropinirole or salts thereof optionally with other pharmaceutically acceptable excipients and b) an outer tablet of other pharmaceutically acceptable excipients. The invention also provides an extended release pharmaceutical composition comprising a core of ropinirole or salt thereof and outer mantle coating comprising one or more pharmaceutically acceptable excipients. The invention also relates to processes of preparing such compositions.

### Background of the Invention

Ropinirole is an orally administered non-ergoline dopamine agonist. It is available as the hydrochloride salt of 4-[2-(dipropylamino)ethyl]-1,3-dihydro-2H-indol 2-one monohydrochloride of formula 1. It is commercially marketed under the trade name of Requip^{®} and Requip XL^{®}. Ropinirole is indicated for the treatment of the signs and symptoms of idiopathic Parkinson's disease and treatment of moderate-to-severe primary Restless Legs Syndrome (RLS).

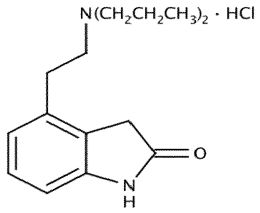

FORMULA 1

U.S. Patent No. 4,452,808 discloses a compound ropinirole and its pharmaceutically acceptable salt forms.

U.S. Patent No. 5,422,123 discloses a Geomatrix system for the controlled-rate release of active substance. Marketed formulation of extended release ropinirole i.e Requip XL^{®} is based on the Geomatrix technology.

USApplication No. 20070264336 discloses multi-layer controlled release tablet, consisting of one active layer and one or more barrier layers containing hydrophillic and lipophilic polymeric substances which limit the release surface of the active layer.

Ropinirole has also been disclosed as being of potential use in the treatment of a variety of other conditions such as Parkinson's disease (U.S. Patent No. 4,824,860); fibromyalgia (U.S. Patent No. 6,277,875); depression (U.S. Patent No. 4,912,126); hypertension, angina pectoris, congestive heart failure or kidney dysfunction (US Patent No. 4,588,740) and chronic fatigue syndrome (U.S. Patent No. 6,300,365).

U.S. Application No. 20070059365 and U.S. Application No. 2003180359 and PCT publication No. WO2006032202 disclose the sustained release dosage forms of ropinirole.

Though there are various techniques known in the art for preparing extended release dosage form of ropinirole, still a need exists for a dosage form that will provide good bioavailability with constant therapeutic concentrations of ropinirole without fluctuating plasma levels.

### Summary of the Invention

In one general aspect of the invention there is provided an extended release tablet in tablet dosage form of ropinirole or salts thereof comprising (a) an inner tablet core of ropinirole or salts thereof optionally with pharmaceutically acceptable excipients; and (b) an outer tablet of pharmaceutically acceptable excipients.

In another general aspect of the invention there is provided an extended release tablet in tablet dosage form of ropinirole or salts thereof comprising (a) an inner tablet core of ropinirole or salts thereof and one or more rate controlling polymers optionally with pharmaceutically acceptable excipients; and (b) an outer tablet of pharmaceutically acceptable excipients.

In another general aspect of the invention there is provided an extended release tablet in tablet dosage form of ropinirole or salts thereof comprising (a) an inner tablet core of ropinirole or salts thereof optionally with one or more rate controlling polymers; and (b) an outer tablet of pharmaceutically acceptable excipients and one or more rate controlling polymers.

In another general aspect of the invention there is provided an extended release tablet in tablet dosage form of ropinirole or salts thereof comprising (a) an inner tablet core of ropinirole or salts thereof with one or more rate controlling polymers; and (b) an outer tablet of pharmaceutically acceptable excipients and one or more rate controlling polymers.

In another general aspect of the present invention there is provided a process for preparing a tablet in tablet dosage form of ropinirole or salts thereof, wherein the process comprises of mixing, granulating ropinirole or salts thereof with one or more rate controlling polymers optionally with other pharmaceutically acceptable excipients to form a core tablet; and compressing pharmaceutically acceptable excipients onto said core tablet.

In another general aspect of the invention there is provided an extended release pharmaceutical composition of ropinirole or salts thereof comprising a core of ropinirole or salts thereof and outer mantle coating surrounding the core comprising one or more pharmaceutically acceptable excipients.

In another general aspect of the invention there is provided an extended release pharmaceutical composition of ropinirole or salts thereof comprising a core of ropinirole or salts thereof, one or more rate controlling polymers and outer mantle coating surrounding the core comprising one or more pharmaceutically acceptable excipients optionally with one or more rate controlling polymers.

In another general aspect of the invention there is provided an extended release pharmaceutical composition of ropinirole or salts thereof comprising a core of ropinirole or salts thereof, optionally with one or more rate controlling polymers and outer mantle coating surrounding the core comprising one or more pharmaceutically acceptable excipients and one or more rate controlling polymers.

In another general aspect of the invention there is provided an extended release pharmaceutical composition of ropinirole or salts thereof comprising a core of ropinirole or salts thereof, with one or more rate controlling polymers and outer mantle coating surrounding the core comprising one or more pharmaceutically acceptable excipients and one or more rate controlling polymers.

In another general aspect of the invention there is provided a process for preparing an extended release pharmaceutical composition of ropinirole or salts thereof, which process comprises of mixing, granulating ropinirole or salts thereof optionally with one or more rate controlling polymers to form a core; and forming an outer mantle coating of pharmaceutically acceptable excipients optionally with one or more rate controlling polymers surrounding the core.

In another general aspect of the invention, there is provided an extended release pharmaceutical composition of ropinirole or salts thereof, comprising a core of ropinirole or salts thereof and outer mantle coating surrounding the core comprising one or more pharmaceutically acceptable excipients, wherein the composition exhibits a dissolution profile such that at least 5% of ropinirole is released within 1 hour; at least 10% of ropinirole is released within 2 hours; at least 35% of ropinirole is released within 6 hours; at least 50% of ropinirole is released within 12 hours.

Embodiments of the pharmaceutical composition may include one or more of the following features. For example, the pharmaceutically acceptable excipients may include one or more of fillers, binders, lubricants, sweeteners, coloring and flavoring agents, glidants, disintegrants, and the like.

The details of one or more embodiments of the inventions are set forth in the description below. Other features, objects and advantages of the inventions will be apparent from the description and claims.

### Detailed Description of the Invention

The inventors have now discovered that extended release form of ropinirole providing release profile maintaining constant plasma levels of ropinirole over an extended period of time can be made without using Geomatrix technology. The dosage form of the invention is a tablet in tablet dosage form comprising an inner tablet core of ropinirole or salts and an outer tablet of pharmaceutically acceptable excipients. The dosage form of the invention also involves a mantle coating of pharmaceutically acceptable excipients over ropinirole core. The said mantle coating completely envelops the core. The composition of the invention exhibits release profile maintaining constant plasma levels of ropinirole over an extended period of time.

The term 'mantle coating' as used herein refers to an outer coat comprising one or more excipients, which completely envelops the ropinirole core.

The outer mantle coating may optionally comprise ropinirole or salts thereof.

The outer tablet or outer coating may further comprise ropinirole or salts thereof.

The term 'extended release' as used herein may interchangeably be used with prolonged release, modified release, controlled release, slow release and sustained release.

Suitable rate controlling polymers may be selected from a group comprising of one or more of carbohydrate gum, polyuronic acid salts, cellulose ethers, acrylic acid polymers and mixtures, natural or synthetic waxes, fatty acids, fatty alcohols, fatty acid esters, fatty acid glycerides including mono-, di-and tri-glyceride, hydrocarbons, hydrogenated fats, hydrogenated castor oils and hydrogenated vegetable oils, can be used. Said melt granulating agent, though not particularly limited, include cetostearyl alcohol, stearyl alcohol, cetyl alcohol, myristyl alcohol and lauryl alcohol, hydrogenated vegetable oil, glyceryl monostearate, glycerol monooleate, acetylated mono glyceride, tristearin, tripalmitin, cetyl ester wax, glyceryl palmitostearate and glyceryl behenate, microcrystalline wax, beeswax, carnauba wax, glyco wax and castor wax.

Suitable carbohydrate gums include one or more of xanthan gum, tragacanth gum, gum karaya, guar gum, acacia, gellan, locust bean gum, ghatti gum, agar, gum arabic, galactomannan, irish moss, carrageenan xylogalactan, glucomannan and other carbohydrate gums having similar properties. Suitable polyuronic acid salts include one or more of alkali metal salts of alginic acid or pectic acid and mixtures thereof.

Suitable alkali metal salts of alginic acid that may be used include one or more of sodium alginate, potassium alginate, ammonium alginate, propylene glycol alginate and other suitable alkali metal salts of alginic acid.

Suitable cellulose ethers include one or more of methylhydroxypropylcelluloses, hydroxyethylcelluloses, hydroxypropylcelluloses, methylcelluloses, ethyl celluloses and carboxymethylcelluloses and other suitable cellulose ethers.

Suitable acrylic acid polymers include any suitable polyacrylic acid polymers or carboxyvinyl polymers such as those available under the brand name carbopol. Pharmaceutically acceptable acrylic polymer may be include one or more, but not limited to acrylic acid and methacrylic acid copolymers, methyl methacrylate copolymers, ethoxyethyl methacrylates, cynaoethyl methacrylate, aminoalkyl methacrylate copolymer, poly(acrylic acid), poly(methacrylic acid), methacrylic acid alkylamide copolymer, poly (methyl methacrylate), poly (methacrylic acid) (anhydride), methyl methacrylate, polymethacrylate, poly(methyl methacrylate), poly (methyl methacrylate) copolymer, polyacrylamide, aminoalkyl methacrylate copolymer, poly(methacrylic acid anhydride), and glycidyl methacrylate copolymers.

Pharmaceutically acceptable excipients may include one or more of diluent, filler, binder, lubricant, glidant and the like.

Suitable fillers may be one or more oflactose, lactose monohydrate, mannitol, sucrose, maltodextrin, dextrin, maltitol, sorbitol, xylitol, powdered cellulose, cellulose gum, microcrystalline cellulose, starch, calcium phosphate, metal carbonate and the like.

Suitable binders may be one or more of starch, sugars, gums, low molecular weight,polyvinylpyrrolidone and hydroxypropyl cellulose and the like.

Suitable lubricants may be one or more talc, magnesium stearate, polyethylene glycol, hydrogenated vegetable oils, stearic acid, sodium stearyl fumarate and sodium benzoate and the like.

Suitable glidants may be one or more of colloidal silicon dioxide, talc, magnesium stearate and the like. Suitable disintegrant may be one or more of starch, croscarmellose sodium, crospovidone, sodium starch glycolate and the like.

The pharmaceutical dosage form may be prepared by various techniques known in the art such as direct compression, dry granulation or wet granulation.

The tablet-in-tablet dosage form of the invention may be prepared by compressing ropinirole or salts thereof optionally with pharmaceutically acceptable excipients to form a core tablet; and compressing pharmaceutically acceptable excipients onto said core tablet to form a compressed outer tablet. The outer tablet may optionally comprise ropinirole or salt thereof.

The extended release composition of the invention may also be prepared by compressing ropinirole or salt thereof optionally with one or more pharmaceutically acceptable excipients to form a core and forming outer mantle coating with one or more pharmaceutically acceptable excipients.

While the present invention has been described in terms of its specific embodiments, certain modifications and equivalents will be apparent to those skilled in the art and are intended to be included within the scope of the present invention.

**Examples** -The dosage form of batches is provided in following tables 1 to 5. Following dosage forms are representatives of the preferred dosage forms of the invention. The preparation of example is detailed below.

Example - 1

[Table 1] [Table ]

**Table 1 - Dosage form of Ropinirole tablets**

| **Ingredients** | **%w/w** |
|---|---|
| Inner Tablet | |
| Ropinirole HCl | 0.20-5.0 |
| Hypromellose | 20-70 |
| Carboxymethylcellulose sodium | 2-20 |
| Maltodextrin | 1-10 |
| Lactose Monohydrate | 10-60 |
| Hydrogenated Castor Oil | 5-30 |
| Magnesium Stearate | 0.25-2 |
| Colloidal Silicon Dioxide | 0.25-2 |
| Outer Tablet | |
| Hypromellose | 40-80 |
| Mannitol | 10-40 |
| Povidone | 2-10 |
| Yellow Iron Oxide | 0.25-1 |
| Magnesium Stearate | 0.25-2 |
| Colloidal Silicon Dioxide | 0.25-2 |

Procedure: Ropinirole hydrochloride,hypromellose, carboxymethylcellulose sodium, hydrogenated castor oil and lactose monohydrate were mixed properly. This blend was further mixed with maltodextrin and colloidal silicon dioxide in blender and further lubricated with magnesium stearate. This blend was compressed into suitable dosage form. Further other excipients were compressed using suitable tooling so that inner tablet is placed as inner core in the outer tablet.

Table 2 -Dissolution data of the tablets prepared as per example 1.

**[Table 2] [Table ]**

| **Time (Hrs)** | **% Drug Released** |
|---|---|
| 0 | 0 |
| 1 | 4 |
| 2 | 4 |
| 4 | 5 |
| 6 | 5 |
| 9 | 9 |
| 12 | 17 |
| 16 | 33 |
| 20 | 51 |

Table 2 provides dissolution data of the Ropinirole tablets prepared as per the formulas provided in table 1. For determination of drug release rate, USP Type 2 Apparatus with 10# sinkers (rpm 100) was used wherein 500 ml of pH 4-citrate buffer at 37°C ± 0.5°C was used as medium.

Example - 2

Table 3 - Composition of Ropinirole tablets

**[Table 3] [Table ]**

| **Ingredients** | **%w/w** |
|---|---|
| Inner Tablet | |
| Ropinirole HCl | 0.20-5.0 |
| Hypromellose | 20-70 |
| Carboxymethylcellulose Sodium | 2-20 |
| Maltodextrin | 1-10 |
| Lactose Monohydrate | 10-60 |
| Hydrogenated Castor Oil | 5-30 |
| Magnesium Stearate | 0.25-2 |
| Colloidal Silicon Dioxide | 0.25-2 |
| Outer Coating | |
| Hypromellose | 40-80 |
| Mannitol | 10-40 |
| Povidone | 2-10 |
| Yellow Iron Oxide | 0.25-1 |
| Magnesium Stearate | 0.25-2 |
| Colloidal Silicon Dioxide | 0.25-2 |

Procedure: Ropinirole hydrochloride,hypromellose, carboxymethylcellulose sodium, hydrogenated castor oil and lactose monohydrate was mixed properly. This blend was further mixed with maltodextrin and colloidal silicon dioxide in blender and further lubricated with magnesium stearate. The lubricated blend was converted into suitable core and further providing outer mantle coating by compressing the other excipients on to said core.

Example - 3

Table 4 - Composition of Ropinirole tablets

**[Table 4] [Table ]**

| **Ingredients** | **%w/w** |
|---|---|
| **Core** | |
| Ropinirole HCl | 0.20-5.0 |
| Pregelatinized Starch | 5-50 |
| Maltodextrin | 1-10 |
| Hypromellose | 20-70 |
| Carboxymethylcellulose Sodium | 2-20 |
| Hydrogenated Castor Oil | 5-30 |
| Povidone | 0.5-10 |
| Colloidal Silicon Dioxide | 0.25-2 |
| Magnesium Stearate | 0.25-2 |
| Outer Coating | |
| Opadry 03B84727 Pink | 1-6 |
| Ethyl Cellulose | 5-30 |
| Methylene Chloride | q.s. |
| Isopropyl Alcohol | q.s. |

Table 5 -Dissolution data of the tablets prepared as per example 3.

**[Table 5] [Table ]**

| **Time (Hrs)** | **% Drug Released** |
|---|---|
| 0 | 0 |
| 1 | 9 |
| 2 | 19 |
| 4 | 32 |
| 6 | 43 |
| 9 | 55 |
| 12 | 65 |
| 16 | 76 |
| 20 | 84 |

Table 5 provides dissolution data of the Ropinirole tablets prepared as per the formulas provided in table 4. For determination of drug release rate, USP Type 2 Apparatus with 10# sinkers (rpm 100) was used wherein 500 ml of pH 4-citrate buffer at 37°C ± 0.5°C was used as medium.

While the present invention has been described in terms of its specific embodiments, certain modifications and equivalents will be apparent to those skilled in the art and are intended to be included within the scope of the invention.

## Claims

1. An extended release pharmaceutical composition of ropinirole or salts thereof, comprising a core of ropinirole or salts thereof and outer mantle coating surrounding the core comprising one or more pharmaceutically acceptable excipients.

2. An extended release pharmaceutical composition of ropinirole or salts thereof according to claim 1, the core further comprising one or more rate controlling polymers and the outer mantle coating surrounding the core comprising one or more pharmaceutically acceptable excipients optionally with one or more rate controlling polymers.

3. The pharmaceutical composition as claimed in claim 2, wherein the rate controlling polymers comprises one or more of carbohydrate gum, polyuronic acid salts, cellulose ethers, acrylic acid polymers and mixtures and waxes.

4. An extended release pharmaceutical composition of ropinirole or salts thereof according to claim 1, comprising a core of ropinirole or salts thereof, optionally with one or more rate controlling polymers, wherein the outer mantle coating surrounding the core comprising one or more pharmaceutically acceptable excipients further comprises one or more rate controlling polymers.

5. The pharmaceutical composition as claimed in claim 4, wherein the rate controlling polymers comprises one or more of carbohydrate gum, polyuronic acid salts, cellulose ethers, acrylic acid polymers and mixtures and waxes.

6. The pharmaceutical composition according to any of the preceding claims, further comprising ropinirole or a salt thereof in the outer mantle coating.

7. A process of preparation of extended release pharmaceutical composition of ropinirole or salts thereof, which process comprises of mixing, granulating ropinirole or salts thereof optionally with one or more rate controlling polymers to form a core; and forming an outer mantle coating surrounding the core optionally with one or more rate controlling polymers.

8. The process of claim 7, wherein the rate controlling polymers comprises one or more of carbohydrate gum, polyuronic acid salts, cellulose ethers, acrylic acid polymers and mixtures and waxes.
